(19) Europäisches Patentamt / European Patent Office / Office européen des brevets

(11) **EP 2 106 747 B1**

(12) **FASCICULE DE BREVET EUROPEEN**

(45) Date de publication et mention
de la délivrance du brevet:
**08.03.2017 Bulletin 2017/10**

(51) Int Cl.:
*A61B 5/00* (2006.01) *A61B 5/11* (2006.01)
*A61B 5/103* (2006.01)

(21) Numéro de dépôt: **09290071.1**

(22) Date de dépôt: **30.01.2009**

(54) **Dispositif d'analyse de la marche**

Ganganalysevorrichtung

Device for analysing walking

(84) Etats contractants désignés:
**DE FR GB IT**

(30) Priorité: **04.02.2008 FR 0800571**

(43) Date de publication de la demande:
**07.10.2009 Bulletin 2009/41**

(73) Titulaire: **Commissariat à l'Énergie Atomique
et aux Énergies Alternatives
75015 Paris (FR)**

(72) Inventeur: **Bonnet, Stéphane
69003 Lyon (FR)**

(74) Mandataire: **Gevers & Orès
41 avenue de Friedland
75008 Paris (FR)**

(56) Documents cités:
**EP-A1- 1 985 233     FR-A- 2 895 499**

- **HELIOT R ET AL: "Continuous identification of gait phase for robotics and rehabilitation using microsensors" ADVANCED ROBOTICS, 2005. ICAR '05. PROCEEDINGS., 12TH INTERNATIONAL CO NFERENCE ON SEATLE, WA, USA JULY 18-20, 2005, PISCATAWAY, NJ, USA,IEEE, 18 juillet 2005 (2005-07-18), pages 686-691, XP010835347 ISBN: 978-0-7803-9178-9**
- **DATABASE COMPENDEX [Online] ENGINEERING INFORMATION, INC., NEW YORK, NY, US; avril 2007 (2007-04), YUN X ET AL: "Self-contained position tracking of human movement using small inertial/magnetic sensor modules" XP002505760 Database accession no. E20074810946636 & PROCEEDINGS - IEEE INTERNATIONAL CONFERENCE ON ROBOTICS AND AUTOMATION 2007 IEEE INTERNATIONAL CONFERENCE ON ROBOTICS AND AUTOMATION, ICRA'07 2007 INSTITUTE OF ELECTRICAL AND ELECTRONICS ENGINEERS INC. US, 2007, pages 2526-2533,**
- **DATABASE INSPEC [Online] THE INSTITUTION OF ELECTRICAL ENGINEERS, STEVENAGE, GB; 7 mars 2007 (2007-03-07), O'DONOVAN K J ET AL: "An inertial and magnetic sensor based technique for joint angle measurement" XP002505761 Database accession no. 9633997 & Journal of Biomechanics Elsevier Science Ltd. UK, vol. 40, no. 12, 7 mars 2007 (2007-03-07), pages 2604-2611, ISSN: 0021-9290**
- **KEMP B ET AL: "Body position can be monitored in 3D using miniature accelerometers and earth-magnetic field sensors" ELECTROENCEPHALOGRAPHY AND CLINICAL NEUROPHYSIOLOGY/ELECTROMYOGRAPHY AND MOTOR CONTROL, ELSEVIER, vol. 109, 1 décembre 1998 (1998-12-01), pages 484-488, XP002367000 ISSN: 0924-980X**

# Description

L'invention porte sur un dispositif d'analyse de la marche d'une personne, et plus précisément sur un dispositif permettant d'identifier des instants et des phases caractéristiques de la marche de cette personne.

En particulier, un dispositif selon l'invention permet de déterminer de manière automatique les phases d'appui et de vol pour chaque foulée de la marche. Accessoirement, il permet le comptage du nombre de foulées et, de ce fait, l'estimation de la cadence de la marche.

Ces informations présentent un intérêt médical et paramédical, notamment en podologie, pour l'analyse descriptive clinique de la marche et pour permettre le déclenchement d'une stimulation électrique fonctionnelle.

L'article de K. Aminian et al. « Spatio-temporal parameters of gait measured by an ambulatory system using miniature gyroscopes », Journal of Biomechanics 35 (2000), pages 689 - 699, décrit un procédé d'analyse de la marche exploitant au moins un gyroscope fixé à un segment tibial d'une personne. Une comparaison avec des signaux acquis grâce à des interrupteurs sensibles à la pression, fixés au niveau du talon et de la pointe du premier (ou gros) orteil, montre que la mesure de vitesse angulaire du segment tibial, réalisée au moyen du gyroscope, permet d'identifier deux instants caractéristiques de la foulée : le moment où la pointe du pied quitte le sol (TO, pour « toe-off ») et celui de pose du talon (HS, pour « heel-strike »). La période comprise entre les instants TO et HS correspond à la phase de vol de la foulée pour la jambe considérée ; inversement, la période comprise entre les instants HS et TO correspond à la phase d'appui.

L'inconvénient du dispositif proposé par K. Aminian et al. est lié à l'utilisation de gyroscopes, qui sont des dispositifs relativement encombrants (et donc peu confortables pour la personne) et présentant une consommation électrique élevée.

L'utilisation d'interrupteurs sensibles à la pression, également divulguée par l'article précité, ne donne des informations que sur la phase d'appui, et pas sur la phase de vol de la foulée.

Le document EP 1 721 573 décrit un procédé d'estimation de la phase d'un mouvement d'un objet, comportant l'acquisition de données expérimentales à partir de mesures de grandeurs physiques par l'intermédiaire d'au moins un capteur. En particulier, ce document évoque la détermination de l'instant de pose du talon (instant HS) par l'analyse d'un signal d'accélération. L'inconvénient principal de ce procédé est sa complexité computationnelle. De plus, les accéléromètres sont sensibles à la combinaison indissociable de l'accélération de pesanteur et de l'accélération du capteur. Cette dernière présente des composantes à haute fréquence, qui rendent délicate la détermination de la direction de l'accélération de pesanteur.

L'article de S. Bonnet et R. Héliot « A Magnetometer-Based Approach for Studying Human Movements », IEEE Transactions on Biomedical Engineering, Vol. 54, No. 7, juillet 2007, pages 1353 - 1355 décrit un procédé permettant d'étudier l'inclinaison du torse à l'aide de magnétomètres. En particulier, en faisant l'hypothèse d'un mouvement planaire, il est possible de déterminer l'angle formé par le torse par rapport à la verticale. Cependant, le traitement des données est complexe (minimisation d'une fonction de coût par rapport à deux variables). En outre, le document ne fournit aucune indication sur comment on pourrait utiliser l'information ainsi obtenue pour identifier des instants et des phases caractéristiques de la marche d'une personne.

Le document FR 2 895 499 décrit un procédé de mesure de l'orientation d'un solide à l'aide d'un magnétomètre, ainsi que son application à la détermination de l'orientation de la cuisse et du tibia d'une jambe au cours de la marche, dont on peut déduire l'angle du genou. L'identification d'instants et/ou de phases caractéristiques de la marche n'est ni décrite, ni suggérée.

L'article de R. Héliot et al. « Continuous Identification of Gait Phase for Robotics and Rehabilitation Using Microsensors », Proceedings of the International Conference on Advanced Robotics, 2005 - ICAR '05, décrit un procédé de détermination des phases de la marche à partir de mesures d'orientation du tibia et de la cuisse, réalisées à l'aide de capteurs respectifs, comportant des accéléromètres et des magnétomètres, procédé qui nécessite d'un traitement des données complexe.

L'invention vise à résoudre au moins certains des inconvénients de l'art antérieur, en fournissant un dispositif d'analyse de la marche simple quant à sa structure matérielle et au procédé de traitement des données qu'il met en oeuvre.

Avantageusement, le dispositif de l'invention est d'utilisation simple et flexible, car il exploite le champ magnétique terrestre, naturellement présent partout. La personne ne doit pas nécessairement marcher dans une direction déterminée, ni même en ligne droite.

Les magnétomètres étant des capteurs de petites dimensions avec une consommation très faible, le port du dispositif selon l'invention ne procure qu'une gêne minime.

Conformément à l'invention, au moins un des buts précité peut être atteint à l'aide d'un dispositif pour l'analyse de la marche d'une personne, caractérisé en ce qu'il comporte :

- un magnétomètre destiné à être fixé à un segment tibial de ladite personne, pour générer un signal représentatif d'au moins une projection dans un plan sagittal d'un champ magnétique ambiant dans lequel il est immergé ; et
- un moyen de traitement du signal, pour identifier des instants et/ou des phases caractéristiques de la marche de ladite personne à partir dudit signal généré par ledit magnétomètre.

Selon des modes de réalisation particuliers de l'invention :

- Ledit moyen de traitement du signal peut être adapté pour identifier des instants caractéristiques de la marche de la personne par repérage de points particuliers de l'expression en fonction du temps dudit signal généré par ledit magnétomètre.
- Plus particulièrement, ledit moyen de traitement du signal peut être adapté pour identifier des instants caractéristiques de la marche de la personne par repérage d'extrema locaux de l'expression en fonction du temps de l'angle formé par ladite projection dans un plan sagittal du champ magnétique ambiant par rapport à la verticale.
- En variante, ledit moyen de traitement du signal peut être adapté pour identifier des instants caractéristiques de la marche de la personne par repérage d'extrema locaux de l'expression en fonction du temps de la dérivée temporelle de l'angle formé, par rapport à la verticale, par ladite projection dans un plan sagittal du champ magnétique ambiant.
- Selon une autre variante, ledit moyen de traitement du signal peut être adapté pour identifier des instants caractéristiques de la marche de la personne par repérage d'extrema locaux de l'expression en fonction du temps d'une composante sensiblement perpendiculaire au segment tibial de la personne de ladite projection dans un plan sagittal du champ magnétique ambiant.
- Ledit magnétomètre peut être un magnétomètre à trois axes.
- Dans ce cas, ledit moyen de traitement peut être également adapté pour déterminer le cap de la marche à partir d'un signal représentatif d'au moins une composante medio-latérale dudit champ magnétique ambiant, également généré par ledit magnétomètre.
- Ledit moyen de traitement peut être également adapté pour recevoir en entrée une information de calibrage indicative de l'inclinaison dudit champ magnétique ambiant par rapport à la verticale, et pour exploiter ladite information de calibrage afin de déterminer l'angle entre ledit segment tibial et l'axe vertical aux instants caractéristiques de la marche de la personne.
- Le dispositif peut comporter également un deuxième magnétomètre, adapté pour être fixé à une cuisse de ladite personne, ledit magnétomètre étant adapté pour générer un deuxième signal représentatif d'au moins une projection dans un plan sagittal d'un champ magnétique ambiant dans lequel il est immergé ; dans lequel ledit moyen de traitement du signal est également adapté pour déterminer un angle de flexion d'un genou de ladite personne à partir du signal généré par lesdits magnétomètres.

L'invention porte également sur l'utilisation d'un dispositif tel que décrit ci-dessus pour identifier des instants et des phases caractéristiques de la marche d'une personne, ladite personne étant immergée dans le seul champ magnétique terrestre et ledit magnétomètre étant fixé à un segment tibial de ladite personne avec une orientation au moins approximativement connue par rapport au plan sagittal de ce dernier.

D'autres caractéristiques, détails et avantages de l'invention ressortiront à la lecture de la description faite en référence aux dessins annexés donnés à titre d'exemple et qui représentent, respectivement :

- la figure 1, un dispositif selon l'invention en situation d'utilisation ;
- la figure 2, les plans anatomiques du corps humain ;
- la figure 3, le champ magnétique terrestre dans le repère géophysique inertiel ;
- les figures 4A er 4B, la projection du champ magnétique terrestre dans le plan sagittal du corps humain de la figure 2 ;
- les figures 5A et 5B, la détermination de l'angle d'inclinaison du magnétomètre (et donc du segment tibial) par rapport à la verticale ; et
- les figures 6A à 6D, les différents signaux générés par le magnétomètre et leur utilisation pour l'identification d'instants et de phases caractéristiques de la marche; et
- la figure 7, l'utilisation d'une mesure de l'angle de flexion du genou pour l'identification d'instants et de phases caractéristiques de la marche.

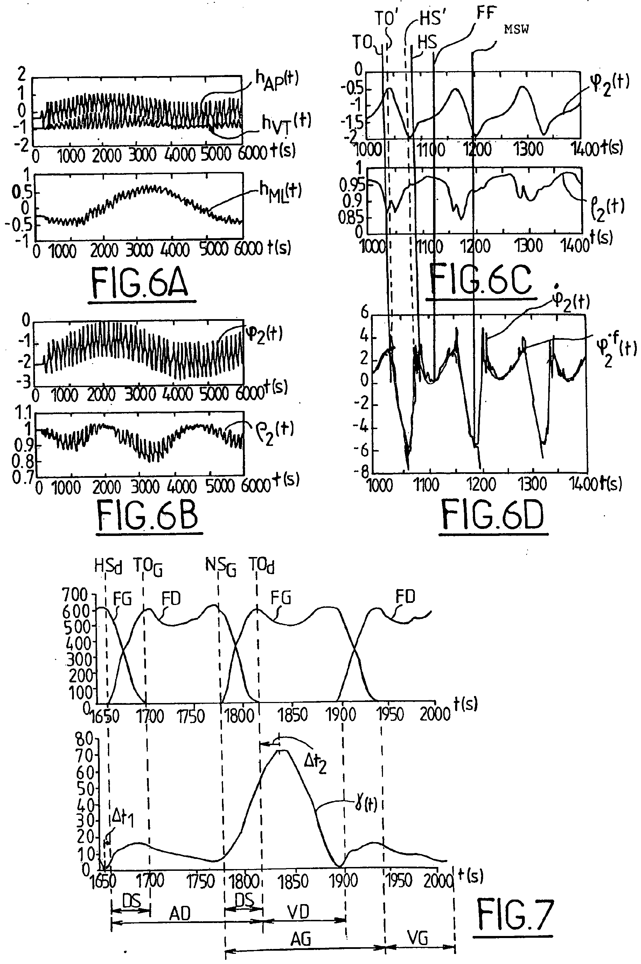

Dans les graphiques des figures 6A à 7, l'axe des abscisses représente le temps, exprimé en secondes. Les unités sur l'axe des ordonnées sont arbitraires.

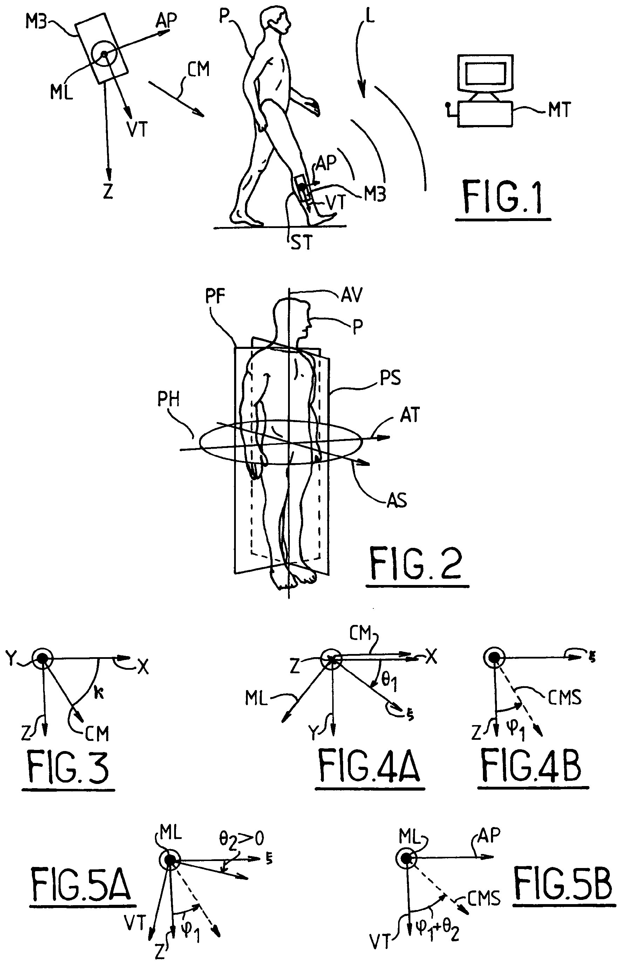

Comme le montre la figure 1, un dispositif selon l'invention comporte un magnétomètre à trois axes M3 pouvant être fixé à un segment tibial ST d'une personne P, ainsi qu'un moyen de traitement du signal relié audit magnétomètre par une liaison L, par exemple sans fils. Dans l'exemple de la figure, le moyen de traitement MT est un ordinateur de bureau, mais il pourrait également s'agir d'un dispositif portatif fixé au corps de la personne ou à ses vêtements. La liaison L assure la transmission au moyen de traitement MT des signaux générés par le magnétomètre M3 qui, tout comme la personne elle-même, se trouve immergé dans un champ magnétique ambiant, typiquement le champ magnétique terrestre, représenté par le vecteur CM sur la figure. Cette liaison L ne doit pas nécessairement être réalisée en temps réel : il est possible de prévoir un moyen d'enregistrement des signaux du magnétomètre pour effectuer une analyse « hors-ligne ».

Les axes de mesure du magnétomètre M3, supposés orthogonaux entre eux, sont indiqués par les références VT, AP et ML. L'axe VT (pour « vertical ») est parallèle au segment tibial, orienté vers le bas ; malgré son nom, il ne reste pas vertical lorsque la personne marche. L'axe AP (pour « antéropostérieur ») est orienté vers l'avant, tandis que l'axe ML (medio-latéral) est orienté latéralement. Au cours de la marche, on peut supposer que l'axe ML reste horizontal et gît dans le plan frontal PF du corps de la personne, tandis que les axes AP et VT tournent dans le plan sagittal PS.

Les plans anatomiques corps humain (plan horizontal, PH ; plan frontal PF ; plan sagittal PS), ainsi que les axes vertical AV, sagittal AS et transverse AT sont représentés sur la figure 2.

L'invention n'est pas limitée au cas où le champ magnétique ambiant est le champ magnétique terrestre ; cependant, il s'agit là d'un mode de réalisation préféré de l'invention, car ce champ magnétique est présent partout, est sensiblement uniforme à l'échelle de mise en oeuvre de l'invention et son inclinaison est au moins approximativement connue.

La figure 3 représente le vecteur du champ magnétique terrestre, CM, dans un repère inertiel orthonormal XYZ où :

- l'axe Z pointe vers le bas (verticale locale) ;
- l'axe X est horizontal et pointe vers le nord magnétique ;
- l'axe Y est horizontal et pointe vers l'est.

Le champ magnétique CM pointe vers le nord magnétique (par définition de ce dernier), et forme un angle $\kappa$ avec l'axe X. L'inclinaison $\kappa$ du champ magnétique CM par rapport à l'horizontale dépend du lieu (elle vaut environ 60° en France), mais peut être considérée comme localement constante. En supposant sa magnitude normalisée à 1, le champ magnétique CM s'exprime, dans le repère inertiel XYZ : $h_i = \begin{pmatrix} \cos \kappa \\ 0 \\ \sin \kappa \end{pmatrix}$

Le champ magnétique CM peut également être exprimé dans le repère mobile du capteur (AP, ML, VT). Sur les figures 4A et 4B, le vecteur $\xi$ indique la direction de la marche, caractérisé par un azimut (par rapport au nord magnétique) $\theta_1$, et le plan $(Z,\xi)$ coïncide avec le plan sagittal PS du corps. Idéalement, l'axe AP du magnétomètre est aligné avec la direction de marche $\xi$.

Sur la figure 4B, le vecteur CMS représente la projection dans le plan sagittal $(Z, \xi)$ du champ magnétique CM. Ce vecteur forme un angle $\varphi_1$ avec la verticale (axe Z). Il est important de noter qu'en général $\varphi_1 \neq \kappa$.

Le vecteur CMS peut s'exprimer, dans le plan $(Z,\xi)$, par $\begin{pmatrix} \sin \kappa \\ \cos \theta_1 \cos \kappa \end{pmatrix} = \rho_1 \begin{pmatrix} \cos \varphi_1 \\ \sin \varphi_1 \end{pmatrix}$. La norme $\rho_1$ de ce vecteur dépend aussi bien de $\kappa$ que du cap de la marche, $\theta_1$.

Lors de la marche, les axes AP et VT du magnétomètre tournent dans le plan sagittal, alors que l'axe ML reste fixe et perpendiculaire à ce plan. Soit $\theta_2$ l'angle d'inclinaison du segment tibial de la personne par rapport à la verticale ou, de manière équivalente, l'angle compris entre l'axe VT et la verticale, ou l'angle compris entre l'axe AP et l'horizontale. On prend la convention selon laquelle l'angle $\theta_2$ est positif lorsque le segment tibial est orienté vers l'arrière (c'est à dire, notamment, à l'instant « TO » où la pointe du pied quitte le sol).

D'une manière tout à fait générale, le vecteur CMS (projection dans le plan sagittal du champ magnétique terrestre) peut être exprimé par ses composantes selon les axes du magnétomètre, $h_{AP}$ et $h_{VT}$, ou en coordonnées polaires $(\rho_2, \varphi_2)$ : $\begin{pmatrix} h_{VT} \\ h_{AP} \end{pmatrix} = \rho_2 \begin{pmatrix} \cos \varphi_2 \\ \sin \varphi_2 \end{pmatrix}$.

La phase $\varphi_2$ de l'expression du vecteur CMS en coordonnées polaires est définie de telle manière que $\varphi_2 = 0$ lorsque ce dernier est orienté parallèlement à l'axe du segment tibial, c'est à dire lorsque le vecteur CMS est parallèle à l'axe VT. En d'autres termes, $\varphi_2$ représente l'angle formé par la projection dans le plan sagittal PS du champ magnétique ambiant CM par rapport à l'axe VT.

Les figures 5A et 5B montrent que :

$$\begin{cases} \rho_2 = \rho_1 \\ \varphi_2 = \theta_2 + \varphi_1 \end{cases}$$

La deuxième de ces équations montre que la phase $\varphi_2$ est égale à l'angle du segment tibial $\theta_2$ plus un décalage $\varphi_1$ qui dépend de $\kappa$ et du cap de la marche $\theta_1$. Comme $\kappa$ est localement uniforme, si la marche s'effectue en ligne droite (cap constant), le décalage $\varphi_1$ est constant.

Une idée à la base de l'invention est que la connaissance de l'angle $\varphi_2$, et de sa variation dans le temps, permet de déterminer les instants et les phases caractéristiques de la marche, et cela même si la marche ne s'effectue pas à cap constant.

La figure 6A montre les graphiques des trois composantes du champ magnétique CM mesuré par le magnétomètre M3 dans le cas d'une marche circulaire : $h_{AP}(t)$, $h_{VT}(t)$ sur le diagramme supérieur, $h_{ML}(t)$ sur le diagramme inférieur. Théoriquement, dans ces conditions, $h_{ML}(t)$ devrait suivre une loi sinusoïdale. Les composantes à haute fréquence visibles sur la figure sont dues à un alignement imparfait de l'axe ML du magnétomètre avec l'axe transverse de la personne.

La figure 6B montre les graphiques des composantes polaires du vecteur CMS : sur le diagramme supérieur, la phase $\varphi_2(t)$ ; sur le diagramme inférieur, la norme $\rho_2(t)$. Comme expliqué plus haut, ces grandeurs peuvent être obtenues à partir des valeurs mesurées $h_{AP}(t)$, $h_{VT}(t)$ grâce à l'équation précitée

$$\begin{pmatrix} h_{VT} \\ h_{AP} \end{pmatrix} = \rho_2 \begin{pmatrix} \cos\varphi_2 \\ \sin\varphi_2 \end{pmatrix}.$$

La figure 6C constitue une vue de détail de la figure 6B.

La figure 6D montre le graphique de la dérivée temporelle de $\varphi_2(t)$, indiquée par $\dot{\varphi}_2(t)$, ainsi qu'une version filtrée (passe-bas) de cette dernière $\dot{\varphi}_2^f(t)$. Les graphiques des figures 6C et 6D sont alignés.

On peut observer que les graphiques de $\dot{\varphi}_2(t)$ et $\dot{\varphi}_2^f(t)$ ressemblent étroitement au graphique de vitesse angulaire du segment tibial reproduit dans l'article précité de K. Aminian et al. Il est donc possible d'identifier des points remarquables de la courbe $\dot{\varphi}_2^f(t)$ (plus facilement exploitable que $\dot{\varphi}_2(t)$, car plus lisse) et de les associer aux instants TO et HS qui découpent le cycle de marche. Ces points remarquables sont les points de maximum de $\dot{\varphi}_2^f(t)$ ; si une convention de signe opposée avait été choisie, ce seraient des points de minimum.

Comme les maxima de $\dot{\varphi}_2^f(t)$ sont très étroits, les points de maximum sont temporellement très proches des passages pas zéro, qui à leur tour correspondent aux points extrêmales de la courbe représentant $\varphi_2(t)$. Par conséquent, comme montré sur les figures 6C et 6D, une détermination approchée des instants caractéristiques de la marche, TO' et HS', peut s'effectuer directement en identifiant les extrema de $\varphi_2(t)$. Les instants TO' et HS' présentent un décalage par rapport aux instants TO et HS déterminés à partir du graphique de $\dot{\varphi}_2^f(t)$. Ce décalage peut être estimé par calibrage, mais en règle générale l'utilisation de la dérivée $\dot{\varphi}_2^f(t)$ fournit des résultats plus précis.

D'autres instants remarquables de la phase de marche, l'instant central de la phase oscillante MSW (pour « Mid Swing ») et l'instant où le pied repose à plat sur le sol FF (pour « Foot Flat ») peuvent être identifiés à partir de la courbe $\dot{\varphi}_2^f(t)$. Selon la convention de signe utilisée sur la figure 6D, ces points correspondent aux minima respectivement principal et secondaire de $\dot{\varphi}_2^f(t)$. Contrairement aux instants TO et HS, les instants MSW et FF sont difficiles à identifier sur la courbe $\varphi_2(t)$ de la figure 6C.

Au moment de l'impact du talon sur le sol, la jambe est en quasi-extension, l'angle du genou est donc très faible. Le pied entre en contact (HS), et le genou fléchit pour absorber l'impact jusqu'à la fin de la phase de double appui, le genou atteint un premier maximum local de flexion, le pied est à plat sur le sol (instant de « Foot Flat » : FF). La jambe s'étend ensuite pour permettre la poussée et le passage du corps sur l'autre jambe pour la seconde phase de double appui. Lors de cette seconde phase de double appui, le genou fléchit jusqu'au décollement des orteils (TO) et continue sa flexion pendant le début de la phase oscillante pour permettre le passage vers l'avant du corps. Le genou atteint alors un second maximum local de flexion (instant de « Mid Swing » : MSW)

Lors de l'instant de FF, le pied est appuyé à plat sur le sol, le tibia est orienté verticalement et la vitesse et l'accélération du pied sont nulles à cet instant.

Ainsi, en disposant un dispositif tel que décrit par l'invention sur chaque jambe d'un individu, il est possible de connaître les quatre instants caractéristiques (TO, MSW, FF, HS) correspondant à chaque jambe, en fonction du temps. Cela permet une bonne analyse du modèle de marche.

La recherche des points remarquables de $\varphi_2(t)$ ou de $\dot{\varphi}_2^f(t)$ peut être effectuée de manière automatique, par des techniques connues de l'art antérieur. D'une façon générale, quelle que soit la convention de signe adoptée, on peut déterminer respectivement MSW, FF, TO et HS d'après la courbe $\dot{\varphi}_2^f(t)$, ces points correspondant

- à chaque extremum de plus forte amplitude en valeur absolue (ou extremum principal) $\rightarrow$ MSW ;
- à chaque extremum de même signe qu'un extremum principal, précédent (ou suivant) immédiatement cet extremum principal $\rightarrow$ FF ;
- à l'extremum de signe opposé à l'extremum principal précédent cet extremum principal $\rightarrow$ TO ; et
- à l'extremum de signe opposé à l'extremum principal suivant le dit extremum principal $\rightarrow$ HS.

Cette description est assez universelle dans le cadre d'une marche normale, mais il peut y avoir des variantes pour les marches pathologiques.

La courbe $\dot{\varphi}_2^f(t)$ montre que le cycle de marche est périodique, la période étant définie comme l'écart temporel séparant deux instants caractéristiques identiques successifs.

- L'identification de l'instant FF peut s'avérer particulièrement utile pour déterminer le cap et la trajectoire de la marche. En effet, en correspondance de cet instant, le segment tibial de la jambe est sensiblement vertical. Ainsi, si l'axe VT du magnétomètre est bien aligné avec ledit segment tibial, les deux axes AP et ML gisent dans un plan horizontal et permettent de déterminer le cap de la marche par rapport au nord magnétique par un calcul trigonométrique simple.

En outre, en correspondance de ce même instant FF, l'accélération et la vitesse du segment tibial sont nulles. Si un accéléromètre auxiliaire est prévu au niveau du tibia, cette information vue comme une condition initiale, permet de calculer la trajectoire de la marche par double intégration du signal fourni par ledit accéléromètre.

En variante, il est également possible de déterminer au moins certains points caractéristiques de la marche (HS et TO, plus difficilement MSW et FF) directement à partir du graphique de $h_{AP}(t)$. En effet $h_{AP}(t) \propto \sin\varphi_2 \approx \varphi_2$. La précision de cette méthode alternative est cependant moins bonne. En tout cas, il est préférable de supprimer préalablement la « ligne de base » de la courbe représentant $h_{AP}(t)$, due aux changements de cap de la marche. Cette ligne de base est bien visible sur la figure 6A, où elle a une allure sinusoïdale due au fait que la marche suit une trajectoire circulaire.

En prévoyant un deuxième magnétomètre solidaire de la cuisse de la personne et en appliquant le un traitement du signal équivalent à celui décrit plus haut, il est possible de déterminer l'angle $\psi$ d'inclinaison du fémur de la personne, à moins d'un décalage $\varphi_1$. La soustraction de l'angle $\psi$ du fémur de l'angle $\varphi_2$ du segment tibial donne l'angle de flexion $\gamma$ du genou. Il est intéressant de noter que cette soustraction permet d'éliminer directement le décalage $\varphi_1$.

La mesure de l'angle du genou $\gamma(t)$ présente, en tant que telle, un intérêt médical et paramédical. De plus, la détermination des instants et des phases caractéristiques de la marche peut également se faire à partir de le graphique de $\gamma(t)$, ou de sa dérivée temporelle, comme le montre la figure 7. Cette figure met en relation le graphique de l'angle du genou droit, $\gamma(t)$, avec celui de la composante verticale des forces de réaction au sol (FD et FG pour les pieds droit et gauche respectivement), mesurées à l'aide d'un tapis équipé de capteurs. Les décalages $\Delta t_1$, $\Delta t_2$ entre les « vrais » instants HS et TO et les extrema correspondants de $\gamma(t)$ peuvent être déterminés par calibrage.

Sur la figure 7 sont indiquées les phases de marche délimitées par les instants caractéristiques HS et TO : double support (DS), appui droit (AD), vol droit (VD), appui gauche (AG) et vol gauche (VG).

Un autre paramètre d'intérêt, rendu accessible par l'invention, est l'inclinaison du segment tibial par rapport à la verticale, $\theta_2$. En particulier, il est utile de connaître sa valeur (ou « angle d'attaque ») au moment de pose du talon, HS. Comme $\theta_2 = \varphi_2 - \varphi_1$ et $\varphi_1$ dépend à la fois de $\theta_1$ et de $\kappa$, ce dernier angle étant connu avec une bonne approximation, il faut déterminer le cap $\theta_1$. Une première possibilité consiste à effectuer un calcul trigonométrique sur la composante ML du signal du magnétomètre : $h_{ML} = -\sin\theta_1 \cos\kappa$. Une variante consiste à calculer l'arc-tangente de $h_{ML}/h_{AP}$, en prenant seulement les signaux mesurés pendant la phase d'appui, lorsque le segment tibial est approximativement perpendiculaire par rapport au sol. Cette variante présente l'avantage de ne pas dépendre d'une connaissance précise de l'angle $\kappa$.

## Revendications

1. Dispositif pour l'analyse de la marche d'une personne (P), comportant un magnétomètre ($M_3$) adapté à être fixé à un segment tibial (ST) de ladite personne, et à générer un signal représentatif d'au moins une projection (CMS) dans un plan sagittal (PS) d'un champ magnétique ambiant (CM) dans lequel il est immergé ; **caractérisé en ce qu'**il comporte également un moyen de traitement du signal (MT) configuré pour identifier des instants (HS, TO, MSW, FF) et/ou des phases (DS, AD, VD, AG, VD) caractéristiques de la marche de ladite personne par repérage de points particuliers de l'expression en fonction du temps dudit signal généré par ledit magnétomètre.

2. Dispositif selon la revendication 1, dans lequel ledit moyen de traitement du signal est adapté pour identifier des instants caractéristiques de la marche de la personne par repérage d'extrema locaux de l'expression en fonction du temps de l'angle ($\varphi_2$) formé par ladite projection dans un plan sagittal du champ magnétique ambiant par rapport audit segment tibial.

3. Dispositif selon la revendication 1, dans lequel ledit moyen de traitement du signal est adapté pour identifier des instants caractéristiques de la marche de la personne par repérage d'extrema locaux de l'expression en fonction du temps de la dérivée temporelle ($\dot{\varphi}_2$) de l'angle ($\varphi_2$) formé, par rapport audit segment tibial, par ladite projection dans un plan sagittal du champ magnétique ambiant.

4. Dispositif selon la revendication 1, dans lequel ledit moyen de traitement du signal est adapté pour identifier des instants caractéristiques de la marche de la personne par repérage d'extrema locaux de l'expression en fonction du temps d'une composante ($h_{AP}$) sensiblement perpendiculaire au segment tibial de la personne de ladite projection dans un plan sagittal du champ magnétique ambiant.

5. Dispositif selon l'une des revendications précédentes, dans lequel ledit magnétomètre est un magnétomètre à trois axes.

6. Dispositif selon la revendication 5, dans lequel ledit moyen de traitement est également adapté pour déterminer le cap de la marche ($\theta_1$) à partir d'un signal représentatif d'au moins une composante medio-latérale ($h_{ML}$) dudit champ magnétique ambiant, également généré par ledit magnétomètre.

7. Dispositif selon l'une des revendications précédentes, dans lequel ledit moyen de traitement est également adapté pour recevoir en entrée une information de calibrage indicative de l'inclinaison ($\kappa$) dudit champ magnétique ambiant par rapport à la verticale, et pour exploiter ladite information de calibrage afin de déterminer l'angle entre ledit segment tibial et l'axe vertical auxdits instants caractéristiques de la marche de la personne.

8. Dispositif selon l'une des revendications précédentes comportant également un deuxième magnétomètre, adapté pour être fixé à une cuisse de la personne, ledit magnétomètre étant adapté pour générer un deuxième signal représentatif d'au moins une projection dans un plan sagittal d'un champ magnétique ambiant dans lequel il est immergé ; dans lequel ledit moyen de traitement du signal est également adapté pour déterminer un angle ($\gamma$) de flexion d'un genou de la personne à partir du signal généré par lesdits magnétomètres.

9. Utilisation d'un dispositif selon l'une des revendications précédentes pour identifier des instants (HS, TO, MSW, FF) et des phases (DS, CD, VD, CG, VD) caractéristiques de la marche d'une personne (P), ladite personne étant immergée dans le seul champ magnétique terrestre (CM) et ledit magnétomètre étant fixé à un segment tibial (ST) de la personne avec une orientation au moins approximativement connue par rapport au plan sagittal (PS) de ce dernier.

**Patentansprüche**

1. Vorrichtung zur Analyse des Gangs einer Person (P), aufweisend ein Magnetometer (M3), das ausgebildet ist, um an einem Schienbeinsegment (ST) der Person befestigt zu sein und um ein Signal zu erzeugen, das für mindestens eine Projektion (CMS) in einer sagittalen Ebene (PS) eines umgebenden Magnetfelds (CM) repräsentativ ist, in welches es eingetaucht ist; **dadurch gekennzeichnet, dass** sie ebenfalls ein Signalverarbeitungsmittel (MT) aufweist, das konfiguriert ist, um durch Erfassen besonderer Punkte der Expression in Abhängigkeit von der Zeit des von dem Magnetometer erzeugten Signals Momente (HS, TO, MSW, FF) und/oder Phasen (DS, AD, VD, AG, VD) zu identifizieren, die für den Gang der Person charakteristisch sind.

2. Vorrichtung nach Anspruch 1, wobei das Signalverarbeitungsmittel ausgebildet ist, um charakteristische Momente des Gangs der Person durch Erfassen lokaler Extrema der Expression in Abhängigkeit von der Zeit des Winkels ($\varphi_2$) zu identifizieren, der von der Projektion in einer sagittalen Ebene des umgebenden Magnetfelds in Bezug zum Schienbeinsegment gebildet wird.

3. Vorrichtung nach Anspruch 1, wobei das Signalverarbeitungsmittel ausgebildet ist, um charakteristische Momente des Gangs der Person durch Erfassen lokaler Extrema der Expression in Abhängigkeit von der Zeit der temporären Ableitung ($\dot{\varphi}_2$) des Winkels ($\varphi_2$) zu identifizieren, der von der Projektion in einer sagittalen Ebene des umgebenden Magnetfelds in Bezug zum Schienbeinsegment gebildet wird.

4. Vorrichtung nach Anspruch 1, wobei das Signalverarbeitungsmittel ausgebildet ist, um charakteristische Momente des Gangs der Person durch Erfassen lokaler Extrema der Expression in Abhängigkeit von der Zeit einer zum Schienbeinsegment der Person etwa senkrechten Komponente ($h_{AP}$) der Projektion in einer sagittalen Ebene des umgebenden Magnetfelds zu identifizieren.

5. Vorrichtung nach einem der vorangehenden Ansprüche, wobei das Magnetometer ein Magnetometer mit drei Achsen ist.

6. Vorrichtung nach Anspruch 5, wobei das Verarbeitungsmittel ebenfalls ausgebildet ist, um den Kurs des Gangs ($\theta_1$) aus einem Signal zu bestimmen, das für mindestens eine mediolaterale Komponente ($h_{ML}$) des ebenfalls von dem Magnetometer erzeugten umgebenden Magnetfelds repräsentativ ist.

7. Vorrichtung nach einem der vorangehenden Ansprüche, wobei das Verarbeitungsmittel ebenfalls ausgebildet ist, um eingehend eine Kalibrierinformation über die Neigung ($\kappa$) des umgebenden Magnetfelds in Bezug zur Vertikalen zu empfangen und um die Kalibrierinformation zu verwenden, um den Winkel zwischen dem Schienbeinsegment und der vertikalen Achse zu den charakteristischen Momenten des Gangs der Person zu bestimmen.

8. Vorrichtung nach einem der vorangehenden Ansprüche, aufweisend ebenfalls ein zweites Magnetometer, das ausgebildet ist, um an einem Oberschenkel der Person befestigt zu sein, wobei das Magnetometer ausgebildet ist, um ein zweites Signal zu erzeugen, das für mindestens eine Projektion in einer sagittalen Ebene eines umgebenden Magnetfelds repräsentativ ist, in welches es eingetaucht ist; wobei das Signalverarbeitungsmittel ebenfalls ausgebildet ist, um einen Flexionswinkel ($\gamma$) eines Knies der Person aus dem von den Magnetometern erzeugten Signal zu bestimmen.

9. Verwendung einer Vorrichtung nach einem der vorangehenden Ansprüche, um für den Gang einer Person (P) charakteristische Momente (HS, TO, MSW, FF) und Phasen (DS, CD, VD, CG, VD) zu identifizieren, wobei die Person in das einzige terrestrische Magnetfeld (CM) eingetaucht ist und wobei das Magnetometer an einem Schienbeinsegment (ST) der Person mit einer mindestens ungefähr bekannten Ausrichtung in Bezug zur sagittalen Ebene (PS) desselben befestigt ist.

**Claims**

1. A device for analyzing the gait of a person (P), the device comprising a magnetometer (M3) adapted to be fastened to a tibial segment (ST) of said person, and to generate a signal representative of at least one projection (CMS) onto a sagittal plane (PS) of an ambient magnetic field (CM) in which it is immersed; the device being **characterized in that** it also comprises signal processor means (MT) configured for identifying instants (HS, TO, MSW, FF) and/or phases (DS, AD, VD, AG, VD) that are characteristic of the gait of said person by identifying particular points in the expression as a function of time for said signal generated by said magnetometer.

2. A device according to claim 1, wherein said signal processor means is adapted to identify characteristic instants of the person's gait by identifying local extrema in the expression as a function of time for the angle ($\varphi_2$) formed by said projection onto a sagittal plane of the ambient magnetic field relative to said tibial segment.

3. A device according to claim 1, wherein said signal processor means is adapted to identify characteristic instants of the person's gait by identifying local extrema in the expression as a function of time for the time derivative ($\dot{\varphi}_2$) of the angle ($\varphi_2$) formed, relative to said tibial segment, by said projection onto a sagittal plane of the ambient magnetic field.

4. A device according to claim 1, wherein said signal processor means is adapted to identify characteristic instants of the person's gait by identifying local extrema in the expression as a function of time for a component ($h_{AP}$) that is substantially perpendicular to the person's tibial segment of said projection onto a sagittal plane of the ambient magnetic field.

5. A device according to any preceding claim, wherein said magnetometer is a three-axis magnetometer.

6. A device according to claim 5, wherein said processor means is also adapted to determine the gait heading ($\theta_1$) from a signal representative of at least one medio-lateral component ($h_{ML}$) of said ambient magnetic field, likewise generated by said magnetometer.

7. A device according to any preceding claim, wherein said processor means is also adapted to receive to receive as input calibration information indicative of the inclination ($\kappa$) of said ambient magnetic field relative to the vertical, and to make use of said calibration information in order to determine the angle between said tibial segment and the vertical axis at said characteristic instants of the person's gait.

8. A device according to any preceding claim, also including a second magnetometer adapted to be fastened to a thigh of the person, said magnetometer being adapted to generate a second signal representative of at least one projection onto a sagittal plane of an ambient magnetic field in which it is immersed; wherein said signal processor means is also adapted to determine a flexing angle ($\gamma$) of the person's knee from the signal generated by said magnetometers.

9. The use of a device according to any preceding claim to identify instants (HS, TO, MSW, FF) and phases (DS, CD, VD, CG, VD) that are characteristic of the gait of a person (P), said person being immersed solely in the Earth's magnetic field (CM) and said magnetometer being fastened to a tibial segment (ST) of the person with an orientation that is known at least approximately relative to the sagittal plane (PS) of the person.

EP 2 106 747 B1

FIG.1

FIG.2

FIG.3

FIG.4A

FIG.4B

FIG.5A

FIG.5B

FIG.6A

FIG.6C

FIG.6B

FIG.6D

FIG.7

## RÉFÉRENCES CITÉES DANS LA DESCRIPTION

**Documents brevets cités dans la description**

- EP 1721573 A

- FR 2895499

**Littérature non-brevet citée dans la description**

- **K. AMINIAN et al.** Spatio-temporal parameters of gait measured by an ambulatory system using miniature gyroscopes. *Journal of Biomechanics,* 2000, vol. 35, 689-699
- **S. BONNET ; R. HÉLIOT.** A Magnetometer-Based Approach for Studying Human Movements. *IEEE Transactions on Biomedical Engineering,* Juillet 2007, vol. 54 (7), 1353-1355

- **R. HÉLIOT et al.** Continuous Identification of Gait Phase for Robotics and Rehabilitation Using Microsensors. *Proceedings of the International Conference on Advanced Robotics,* 2005